# EUROPEAN PATENT APPLICATION

(11) **EP 4 488 389 A1**
(43) Date of publication of application: **08.01.2025**
(21) Application number: 23382693.2
(22) Date of filing: 05.07.2023
(51) Int. Cl.: C12Q 1/6886, G01N 33/574

(54) **SIGNATURE FOR THE PROGNOSIS OF BRAIN METASTASIS RELAPSE**

(71) Applicant: Fundación del Sector Público Estatal Centro Nacional de Investigaciones Oncológicas Carlos III (F.S.P. CNIO), 28029 Madrid (ES)
(72) Inventor: ZHU, Lucía, 28029 Madrid (ES); GRAÑA-CASTRO, Osvaldo, 28029 Madrid (ES); VALIENTE CORTÉS, Manuel, 28029 Madrid (ES); SALGADO CRESPO, Irene, 28029 Madrid (ES)
(74) Representative: Clarke, Modet y Cía., S.L.

(57) **Abstract**

The present invention relates to a method for the prognosis of brain metastasis relapse in a subject and to a kit useful in said prognosis.

## Description

### FIELD OF THE INVENTION

The present invention belongs to the field of medicine, particularly to the field of diagnosis or prognosis, more particularly, the present invention relates to a method for the prognosis of brain metastasis relapse and to a kit useful in said prognosis.

### BACKGROUND OF THE INVENTION

Brain metastasis is the most common neurological complication of cancer. When metastatic cells reach the brain, prognosis is poor given that local therapies (i.e., surgery and radiation) have limited benefits for patients and the disease inevitably progresses.

The true prevalence of brain metastasis is not well known, but is probably higher than the available epidemiological estimates (8.5-9.6%), and is certainly more frequent than any other primary tumor in the brain. Upon diagnosis, cancer patients with disease disseminated to their brains continue to face a dismal prognosis, which includes higher morbidity, mortality, and a dramatic increase in the cost of their treatment.

The incidence of brain metastasis continues to increase and yet, current therapies available for patients with disseminated cancer cells in their central nervous system (CNS) have a limited efficacy and fail to improve survival. Consequently, during the past years, there have been recurrent efforts to improve clinical trial design and management specifically concerning this patient population. However, the inclusion of patients with active CNS disease has been limited in the trials of the past, and this represents an unsolved issue.

The rise in the number of patients with brain metastasis is partially due to the increasing number of systemic therapies that work extra-cranially but are unable to provide the same therapeutic benefit in the brain. Consequently, cancer cells present at this secondary site have additional time to evolve and to grow into clinically detectable lesions.

Surgical resection has a well-established role in the management of brain metastasis.

While much effort has been done to determine markers useful for determining the risk of developing brain metastasis from different primary cancers, not much progress has been made in the obtention of tools for analyzing the risk of relapse of a brain metastasis, and in particular of those removed by surgery.

Some representative methods for predicting risk of brain metastasis in patients based on gene signatures, are displayed in the examples below.

US2021371932 provides a lengthy list of markers that might be related to the prognosis of brain metastasis in patients. However, no reference to prognosis of brain metastasis relapse after brain surgery is even suggested.

WO2012031008 also displays a lengthy list of markers which might be relevant in the diagnosis, prognosis and monitoring of a disease or other medical condition. As in the example of above, no reference as to the relevance of the list of markers for the prognosis of brain metastasis relapse after brain surgery is mentioned.

WO2011068839 discloses methods for predicting risk of metastasis and/or cancer recurrence based on gene expression profiling. WO2011068839 discloses gene DPYSL3 in Bladder Cancer Module, Breast Cancer Module 23, Pancreatic Cancer Module 2, Prostate Cancer Module 4, Sarcoma Module 2, Liver Cancer Module 2 and Lymphoma Module 7; and gene LMO7 in Brain Cancer Module 2 and Lung Cancer Module 9. WO2011068839 is silent about brain metastasis relapse.

US2010029748 discloses two sets of genes, one set of 17 genes (table 1) and one set of 18 genes (table 2) that can be used in predicting the risk of cancer metastasis to the brain, and as a screening assay to identify the suitable treatments for brain metastases. Particularly, the genes in table 1 are: overexpression of ANGPTL4, PLOD2, COL13A1, PTGS2, PELI1, MMP1, B4GALT6, HBEGF, CSF3, RGC32, LTBP1, FSCN1, and LAMA4 and underexpression of TNFSF10, RARRES3, SCNN1A and SEPP1 are indicative of an increased likelihood of brain metastases (17 genes set). Also, the genes of table 2 are PCDH7, ARHGDIB, ST6GALNAC5, ODZ3, SERPINI1, GJA1, GARNL4, PRSS3, SERPINE2, KTELC1, RGS2, IL1B, MMP3, TMEM156, FAM59A, IL1A, MOCS1, TLR4.

In US2010029748, gene CYP1B1 is listed in Table 4 as a gene which is differentially expressed by more than 2.5 fold between parental MDA231 and its brain metastatic derivatives, among other 178 genes. Table 3 of US2010029748 discloses another set of 271 genes differentially expressed by more than 2.5 fold between parental CN34 and brain metastatic derivatives. US201 0029748 does not relate to a signature for predicting the risk of relapse or recurrence of brain metastasis.

Kamer et al. (Transl Lung Cancer Res 2020;9(3):682-692) discloses a 22 gene signature for predicting the risk of developing brain metastasis, not for brain metastasis relapse. None of the 22 genes are any of the genes of the signature of the present invention.

Of all the documents of the state of the art, just Soike et al. (Scientific Reports (2019) 9:14385) seems to point to CD138 as a biomarker for recurrence of brain metastasis after surgery.

There is the need to predict the risk of relapse of brain metastasis after treatment and in particular after surgery. The present invention provides a signature that allows the significant determination of the risk of relapse of brain metastasis after treatment and in particular, after surgery.

### DESCRIPTION OF THE INVENTION

The present invention is based on a gene signature for the prognosis of brain metastasis relapse in a patient. The core gene signature is composed of genes LMO7 and/or DPYSL3. The inventors have found that analyzing the expression levels of at least genes LMO7 and/or DPYSL3 in an isolated sample from a subject who underwent treatment, for example brain surgery, constitutes a reliable method for the prognosis of brain metastasis relapse in the subject.

More precisely, the inventors have found that when the expression levels of genes LMO7 and/or DPYSL3 are compared with those of reference populations with and/ or without relapse, an increased expression of gene LMO7 and/or a decreased expression of gene DPYSL3 with respect to a non-relapse reference population, is associated with a higher risk of relapse.

Measurement of the expression levels of the core signature of genes LMO7 and/or DPYSL3 might be combined with measurement of expression levels of additional genes. In particular, with measurement of expression levels of JAGN1.

The signature of the present invention is therefore the basis of a reliable method and kit for the prognosis of brain metastasis relapse after treatment, and more particularly, after brain surgery.

None of the documents of the state of the art disclose or even suggest the relevance of the signature of genes of the present invention for prognosis of brain metastasis relapse in an individual.

The first aspect of the present invention relates to a method for the prognosis of brain metastasis relapse in a subject, wherein the method comprises analyzing the expression levels of at least genes LMO7 and/or DPYSL3 in an isolated sample from the subject.

The method of the present invention is particularly useful for the prognosis of brain metastasis relapse after treatment in a subject, and in particular, after brain surgery.

The second aspect of the present invention relates to a kit comprising means for analyzing the expression levels of genes LMO7 and/or DPYSL3 in a sample.

A third aspect of the present invention relates to the use of the method or of the kit of the present invention for the prognosis of brain metastasis relapse in a subject. And in particular after subjecting the subject to treatment of such brain metastasis, and in particular by brain surgery.

In a preferred embodiment, the expression levels of genes LMO7 and/or DPYSL3 are compared with those of reference populations with and/ or without relapse. And an increased expression of gene LMO7 and/or a decreased expression of gene DPYSL3 when compared with a non-relapse reference population, is associated with a higher risk of relapse.

In another preferred embodiment the method and kit further involve analysis of the expression levels of gene JAGN1. In such a method, a decreased expression of gene JAGN1, when compared with a non-relapse reference population, is associated with a higher risk of relapse.

By detection of expresion levels of a gene it is meant detection of its mRNA levels, but also detection of any corresponding translation product issuing it (eg. the corresponding protein). The gene expression analysis may be performed in a sample by any possible means. Preferably by detection of mRNA levels by qPCR, preferably by RT-qPCR, by *in situ* hybridization, preferably RNAscope, and/ or by protein detection preferably with antibodies.

In another embodiment, the method further comprises analyzing the expression of one or more additional genes, and/ or of one or more reference genes in the sample.

The one or more additional genes can be selected from CYP1 B1 and EDARADD, and from any other set of genes considered to be relevant in relapse of brain metastasis.

In the particular case of genes CYP1B1 and EDARADD, their decreased expression when compared with a non-relapse reference population, is associated with a higher risk of relapse.

Gene LMO7 (LIM domain only protein 7) has Gene ID: 4008, (updated on 8-May-2022) in the NCBI (National Center for Biotechnology Information) Entrez Gene Database. The reference for gene LMO7 in the Ensembl database is ENSG00000136153 and the reference in the Uniprot database is Q8WWI1.

Gene DPYSL3 (dihydropyrimidinase like 3) has Gene ID: 1809, (updated on 24-Apr-2022) in the NCBI (National Center for Biotechnology Information) Entrez Gene Database. The reference for gene DPYSL3 in the Ensembl database is ENSG00000113657 and the reference in the Uniprot database is Q14195. Gene DPYSL3 is also known as TUC4.

Gene JAGN1 (jagunal homolog 1) has Gene ID: 84522, (updated on 8-May-2022) in the NCBI (National Center for Biotechnology Information) Entrez Gene Database. The reference for gene JAGN1 in the Ensembl database is ENSG00000171135 and the reference in the Uniprot database is Q8N5M9.

Gene CYP1B1 (cytochrome P450 family 1 subfamily B member 1) has Gene ID: 1545, (updated on 24-Apr-2022) in the NCBI (National Center for Biotechnology Information) Entrez Gene Database. The reference for gene CYP1B1 in the Ensembl database is ENSG00000138061 and the reference in the Uniprot database is Q16678.

Gene EDARADD (EDAR associated death domain) has Gene ID: 128178, (updated on 24-Apr-2022) in the NCBI (National Center for Biotechnology Information) Entrez Gene Database. The reference for gene EDARADD in the Ensembl database is ENSG00000186197 and the reference in the Uniprot database is Q8WWZ3.

The method and kit might comprise means for analyzing the expression levels of LMO7 and/or DPYSL3, of LMO7, DPYSL3 and JAGN1, of LMO7, DPYSL3, JAGN1 and/or one or both of genes CYP1B1 and EDARADD, of up to 6 genes, of up to 7 genes, of up to 8 genes, of up to 9 genes, of up to 10 genes, of up to 20 genes, of up to 30 genes, of up to 40 genes, of up to 50 genes, of up to 60 genes, of up to 70 genes, of up to 80 genes, of up to 90 genes, of up to 100 genes.

In a preferred embodiment, the reference gene might be selected from actin, beta-2 microglobulin, cytochrome C1, EMG1 N1 -specific pseudouridine methyltransferase, glyceraldehyde-3-phosphate dehydrogenase, TATA-box binding protein, topoisomerase (DNA) I, tyrosine 3- monooxygenase/tryptophan 5-monooxygenase activation protein zeta and combinations thereof, preferably is actin, beta-2 microglobulin or both.

As used herein, the expression "reference population" refers to either a population that has suffered brain metastasis and has not had any relapse after treatment and in particular after brain surgery (non-relapse reference population), or to a population that has suffered brain metastasis, which relapsed after treatment and in particular after brain surgery (relapse reference population).

The sample may be a fluid sample, such as a blood sample or a cerebrospinal fluid sample, or a tissue sample, such as a biopsy, obtained when or after the subject is or has been subjected to treatment, and in particular, to brain metastasis surgery. In a preferred embodiment, the sample is a brain sample obtained by brain surgery of a brain metastasis. In a preferred embodiment, the sample is a cDNA sample (complementary DNA), preferably a cDNA obtained from an RNA sample. As used herein, DNA is Deoxyribonucleic acid and RNA is Ribonucleic acid.

In a preferred embodiment, the subject is human, preferably a human with brain metastasis from a primary cancer, for example, lung cancer, melanoma, colorectal cancer or breast cancer. In an example, the primary cancer is lung cancer, for example, non-small cell lung cancer, and in yet another example, lung adenocarcinoma. In another example, the primary cancer is breast cancer HER positive.

In a preferred embodiment, the gene expression analysis is made by quantitative polymerase chain reaction (qPCR), preferably by RT-qPCR. In another preferred embodiment, the gene expression analysis is made using RNA probes and/or primers.

In a preferred embodiment, the means for analyzing the gene expression comprises a pair of primers for each one of the genes. For example, the following primers can be used for LMO7 and DPYSL3:

| **Gene** | **Sense** | **Sequence** | |
|---|---|---|---|
| LMO7 | Forward | SEQ ID NO: 1 | AGCTTGGACTCTTTGGGCTC |
| | Reverse | SEQ ID NO: 2 | GCAGTCTTTGGCTCAACAGC |
| DPYSL3 | Forward | SEQ ID NO: 3 | CAGCCAAACCAATTGCCCTC |
| | Reverse | SEQ ID NO: 4 | GGGGTGGGGATGTCACAAAT |

And for JAGN1, CYP1B1 and EDARADD:

| **Gene** | **Sense** | **Sequence** | |
|---|---|---|---|
| JAGN1 | Forward | SEQ ID NO: 5 | CACTGTGCCTTGTGTTTGGG |
| | Reverse | SEQ ID NO: 6 | GGCCACTGCTAGGTTTCTCA |
| CYP1B1 | Forward | SEQ ID NO: 7 | AACGTACCGGCCACTATCAC |
| | Reverse | SEQ ID NO: 8 | TGGTAGCCCAAGACAGAGGT |
| EDARADD | Forward | SEQ ID NO: 9 | CCTCCCCTTGTGTCAACTCC |
| | Reverse | SEQ ID NO: 10 | GTGGCTCCGGTGATTTGGG |

In another preferred embodiment, the means for analyzing the gene expression further comprises a pair of primers for at least one reference gene. Preferably, the reference gene or genes is or are selected from actin, beta-2 microglobulin, cytochrome C1, EMG1 N1 - specific pseudouridine methyltransferase, glyceraldehyde-3-phosphate dehydrogenase, TATA-box binding protein, topoisomerase (DNA) I, tyrosine 3- monooxygenase/tryptophan 5-monooxygenase activation protein zeta and combinations thereof, more preferably the reference gene or genes is/ are actin, beta-2 microglobulin or both. Primers for analyzing the gene expression of these reference genes are well known by the skilled person and commercially available.

In a preferred embodiment, the means for analyzing the gene expression further comprises a specific probe for each one of the genes. Preferably, the probe is labeled, preferably duallabeled. As used herein, the term "labeled" means that the probe is marked by means of a fluorescent reporter dye on the 5' base and a quencher located on the 3' base.

In another preferred embodiment, the means further comprises at least one of the following: a DNA polymerase, a nucleotide mix, a buffer, DNase-free water, or any combination thereof. In a preferred embodiment of the present invention, a master mix comprising a DNA-binding dye is comprised.

In another embodiment, the gene expression analysis is made by the quantification of the proteins encoded by the genes of the signature of the present invention, for example by using specific antibodies.

A preferred embodiment of the invention consists of an *in vitro* method for the prognosis of brain metastasis relapse after brain surgery comprising the following steps:
(a) analyzing the expression levels of at least genes LMO7 and/or DPYSL3 in an isolated sample of a subject who has been subjected to brain metastasis surgery, and
(b) comparing the expression levels found in step (a) with those of reference populations with and/or without relapse, and
(c) determining the risk of relapse of the brain metastasis for the subject,
wherein the increased expression of gene LMO7 and the decreased expression of gene DPYSL3, when compared with a non-relapse reference population, is associated with a higher risk of relapse.

The method might additionally include analyzing the expression levels of one or more of genes JAGN1, CYP1B1, EDARADD, wherein the decreased expression of these genes when compared with a non-relapse reference population, is associated with a higher risk of relapse.

A preferred embodiment of the present invention corresponds to a kit comprising means for quantifying the expression of substantially only genes LMO7 and/or DPYSL3, and optionally additionally one or more of genes JAGN1, CYP1B1 and EDARADD. As used herein, the expression "substantially only" means that if any other gene is quantified, then said other gene or genes is or are reference genes, which are only quantified for serving as a reference for the quantification of the expression, as a reference for the sample load. According to this preferred embodiment, an increased expression of gene LMO7 and a decreased expression of genes DPYSL3, JAGN1, CYP1B1 and EDARADD, when compared with a relapse reference population, might be associated with a higher risk of relapse. Contrarily, a decreased expression of gene LMO7 and an increased expression of genes DPYSL3, JAGN1, CYP1B1 and EDARADD, when compared with a relapse reference population, might be associated with a lower risk of relapse.

The present invention also refers to the use of signature of the present invention for deciding or recommending a patient who has suffered from brain metastasis to perform (follow) a treatment to prevent brain metastasis relapse or recommending an alternative medical regime.

The present invention also refers to a method for deciding or recommending a patient who has suffered from brain metastasis a medical regime to prevent brain metastasis relapse after treatment, and in particular after surgery, the method comprising:
a) analyzing the expression levels of at least genes LMO7 and/or DPYSL3, and optionally additionally one or more of genes JAGN1, CYP1B1 and EDARADD in a sample of a subject who has been subjected to brain metastasis treatment and in particular surgery,
b) comparing the expression levels found in step (i) with those of reference populations with and/or without relapse, and
c) recommending a preventive medical regime to prevent brain metastasis relapse in the subject when the expression of gene LMO7 is increased and the expression of genes DPYSL3, JAGN1, CYP1B1 and EDARADD is decreased, in comparison with the non-relapse reference population; or recommending an alternative medical regime if the expression of gene LMO7 is increased and the expression of genes DPYSL3, JAGN1, CYP1B1 and EDARADD is decreased, in comparison with the non-relapse reference population.

The present invention also refers to the use of the signature of the present invention for stratifying brain metastasis patients with regard to their likelihood to develop brain metastasis relapse.

In another embodiment, the method for stratifying brain metastasis patients with regard to their likelihood to develop brain metastasis relapse comprises:
a) analyzing the expression levels of at least genes LMO7 and/or DPYSL3, and optionally additionally one or more of genes JAGN1, CYP1B1 and EDARADD in a sample of a subject who has been subjected to brain metastasis treatment and in particular surgery,
b) comparing the expression levels found in step (i) with those of reference populations with and/or without relapse, and
c) stratifying the likelihood to develop brain metastasis relapse as high if the expression of gene LMO7 is increased and the expression of genes DPYSL3, JAGN1, CYP1B1 and EDARADD is decreased, in comparison with the non-relapse reference population or
d) stratifying the likelihood to develop brain metastasis relapse as low if the expression of gene LMO7 is decreased and the expression of genes DPYSL3, JAGN1, CYP1B1 and EDARADD is increased, in comparison with the relapse reference population.

The present invention also refers to a combined use of an expression product of each one of at least genes LMO7 and/or DPYSL3, and optionally additionally of one or more of genes JAGN1, CYP1B1 and/or EDARADD genes as a marker of prediction of the risk of developing brain metastasis relapse in a subject after treatment and in particular after brain surgery; or of prognosis of brain metastasis relapse after treatment and in particular after brain surgery; or as a marker for deciding or recommending a patient who has suffered from brain metastasis to perform a medical regime to prevent brain metastasis relapse; or for stratifying brain metastasis patients with regard to their likelihood to develop brain metastasis relapse.

All the embodiments of the methods of the present invention are also embodiments of the other aspects of the present invention.

All individual features (e.g. preferred features) mentioned herein may be taken in isolation or in combination with any other feature (including a preferred feature) mentioned herein (hence, preferred features may be taken in conjunction with other preferred features, or independently of them).

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practice of the invention. The following examples and figures are provided for illustrative purposes and are not intended to limit the scope of this invention.

### BRIEF DESCRIPTION OF FIGURES

**Figure 1****.** Representative image of patient-derived brain metastasis matched samples (before and after undergoing neurosurgery), stained with DPYSL3-specific antibody or RNA probe.
**Figure 2****.** Representative image of mouse-derived brain metastasis matched samples, stained with DPYSL3-specific antibody or RNA probe.
**Figure 3****.** Representative image of patient-derived brain metastasis matched samples, stained with LMO7-specific antibody or RNA probe.
**Figure 4****.** Representative image of mouse-derived brain metastasis matched samples, stained with LMO7-specific antibody or RNA probe.
**Figure 5****.** Representative image of patient-derived brain metastasis matched samples, stained with JAGN1 -specific RNA probe.
**Figure 6****.** Representative image of mouse-derived brain metastasis samples, stained with JAGN1 -specific RNA probe.
**Figure 7****.** Results corresponding to analysis of *LMO7* (a), *DPYSL3* (*TUC4*) (b) and *JAGN1* (c) in the 8 human samples of Example 3.
**Figure 8****.** Table with Details of the 8 human samples made use of for the analysis of Example 3.
**Figure 9****.** Table displaying protein quantification results corresponding to *LMO7* and *DPYSL3,* obtained with the 8 human samples of Example 3 (before and after undergoing relapse).

### EXAMPLES

### Material and methods

Established H2030-BrM (Luciferase+/ GFP+ (Green Fluorescent Protein)) metastases were obtained from 6 individual mice (nude) previously implanted intracranially with the human cell line. The initial brain metastasis was obtained using neurosurgery and mice were followed over time until relapse was confirmed using bioluminescence imaging (Living Image software, version 4.5). Mice were sacrificed at endpoint (approximately 6 weeks post-surgery) and the relapsed tumor was microdissected. Both the initial brain metastasis and the relapsed one from the same mice (matched samples) were profiled with RNAseq.

### Animal studies

All animal experiments were performed in accordance with a protocol approved by the CNIO (IACUC.030-2015), institute de Salud Carlos III (CBA35_2015-v2), and Comunidad de Madrid Institutional Animal Care and Use Committee (PROEX250/15 and PROEX135/19). Females athymic nu/nu (Harlan) 4-10 weeks of age were used. Housing and husbandry conditions were in accredited by Association for Assessment and Accreditation of Laboratory Animal Care (AAALAC). Mice were SPF (specific pathogen-free) with microbiological and environmental parameters constantly monitored. Brain colonization assays were performed by injecting 40,000 cancer cells intracranially (the right frontal cortex, approximately 1.5 mm lateral and 1 mm caudal flow bregma, and to a depth of 1 mm) by using a gas-tight Hamilton syringe and a stereotactic apparatus. Brain colonization was analyzed *in vivo* and *ex vivo* by BLI. Anesthetized mice (isoflurane) were injected retro-orbitally with Dluciferin (150 mg/kg; Syd Labs) and imaged with an IVIS machine (Perkin Elmer). Bioluminescence analysis was performed using Living Image software, version 4.5. Brain tumor resection was performed by adapting previously described procedures (Morrissy et al., 2016. Nature 529: 351 -357). In brief, after exposing the skull, a craniotomy was performed surrounding the brain metastasis area, which was visualized by GFP, using an excitation light source at 460-495 nm (FS/ULS-02 B2, BLS Ltd) and goggles carrying emission filters (FHS/EF-3GY1, BLS Ltd). The skull and the dura were lifted with micro-dissecting forceps, and the bulk of the tumor was then removed using a microcurette guided by GFP. When hemostasis was obtained, the surgical wound was sutured using interrupted stitching with absorbable sutures. Animals received meloxicam at 5 mg/kg once per day during 72 h and dexamethasone at 13 mg/kg once per day during 48 h to contain brain edema.

### Cell culture

Human BrM cell line has been previously described and obtained from the same batches that were previously published (Nguyen et al., 2009. Cell 138: 51 - 62). The cell line was validated by morphological analysis and its behavior *in vivo.* The cell line was tested mycoplasma-free. H2030-BrM3 (abbreviated as H2030-BrM) (Origin: Massague lab, Memorial Sloan Kettering Cancer Center) was cultured in RPMI1640 media supplemented with 10% FBS (fetal bovine serum), 2 mM L-glutamine, 100 IU/ml penicillin/ streptomycin, and 1 µg/ml amphotericin B.

### Transcriptomics of relapsed tumors and statistical analysis

500 ng of total RNA samples were used. Sample RNA Integrity numbers were 8.6 on average (range 5.9-9.5) when assayed on an Agilent 2100 Bioanalyzer. Sequencing libraries were prepared with the QuantSeq 3'mRNA-Seq Library Prep Kit (FWD) for Illumina (Lexogen, Cat. No. 015) by following manufacturer's instructions. Library generation was initiated by reverse transcription with oligodT priming, and a second strand synthesis was performed from random primers by a DNA polymerase. Primers from both steps contain Illumina-compatible sequences. Libraries were completed by PCR (This kit generated directional libraries stranded in the sense orientation: the read1, the only read in single read format, had the sense orientation (--library-type fr-secondstrand in TopHat, -- stranded = yes in HTSeq)}. cDNA libraries were purified, applied to an Illumina flow cell for cluster generation and sequenced on an Illumina NextSeq 550 (with v2.5 reagent kits) by following manufacturer's protocols. Eighty-five-base-pair single-end sequenced reads followed adapter and polyA tail removal as indicated by Lexogen. The resulting reads were fed to Xenome to separate the xenograft-derived human and mouse reads. Human reads were analyzed with the nextpresso pipeline as follows: sequencing quality was checked with FastQC v0.11.0. Reads were aligned to the human genome (GRCh38 (Genome Reference Consortium)) with TopHat-2.0.10 using Bowtie 1.0.0 and Samtools 0.1.19, allowing 3 mismatches and 20 multihits. The Gencode v29 gene annotation for GRCh38 was used. Read counts were obtained with HTSeq. Differential expression and normalization were performed with DESeq2, filtering out those genes where the normalized count value was lower than 2 in more than 50% of the samples. From the remaining genes, those that had an adjusted p value below 0.05 FDR were selected. GSEAPreranked was used to perform gene set enrichment analysis for several gene signatures on a pre-ranked gene list, setting 1,000 gene set permutations. Only those gene sets with significant enrichment levels (FDR q-value < 0.25) were considered.

### RNAscope.

Detection of the genes of interest in human and mouse tissue by RNA in situ hybridization. Paraffin-embedded tissue from mouse brain metastases was sectioned and RNAscope 2.5 VS probes for *Hs-DPYSL3* (540289, ACDbio), Hs-*LMO7*(1169419-C1, ACDbio), Hs-*JAGN1* (1169429-C1, ACDbio) were assayed on the Roche Ventana Discovery XT using the standardized automated protocols at the CNIO Histopathology Core Facility.

Antibodies made use of were:
DPYSL3: AB5454, Sigma Aldrich.
LMO7: HPA020923, Sigma Aldrich.

### Results

### Example 1.

H2030-BrM metastasis samples obtained after neurosurgery, as well as samples resulting from relapse (reappearance after surgery), were subjected to RNAseq analysis.

When the original tumors and the relapsed tumors were compared, only 5 genes of the whole transcriptome were significantly deregulated (1 upregulated: LMO7; 4 downregulated: CYP1B1, DPYSL3, EDARADD, JAGN1, in comparison with the initial brain metastasis).

**Table 1. Statistical relevance of the five genes of the signature of the invention.**

| **Gene** | **log2 Fold Change** | **pvalue** | **padj** |
|---|---|---|---|
| CYP1B1 | -3,647148 | 1,40E-18 | 2,16E-14 |
| LMO7 | 2,0089955 | 6,06E-09 | 4,67E-05 |
| DPYSL3 | -1,351161 | 5,76E-08 | 0,000296 |
| EDARADD | -1,146406 | 5,10E-06 | 0,0196618 |
| JAGN1 | -1,918629 | 9,53E-06 | 0,0294155 |

Log2 Fold Change is the result of dividing the FPKM (Fragments per kilo base per million mapped reads) of the second value (relapsed metastasis) on the FPKM of the first value (initial brain metastasis) to get the Fold Change (FC). Then, the log2 fold change value from FPKM value is obtained.

Pvalue and aadi: differential expression and normalization was performed with DESeq2 (Love et al. 2014. Genome Biol. 2014;15(12):550), filtering out those genes where the normalized count value was lower than 2 in more than 50% of the samples. From the remaining genes, those that had an adjusted p-value below 0.05 FDR (False Discovery Rate) were selected.

The main conclusion from the experimental model was that when the expression level of the gene LMO7 is increased and the expression levels of genes CYP1B1, DPYSL3, EDARADD, JAGN1 are decreased comparing to a reference population, then the subject presents a higher risk of relapse.

Confirmation of the relevance of expression levels of genes LMO7 and/or DPYSL3 was achieved both in a lung cancer model and in human brain metastasis samples pre- and post-relapse. Corresponding results are displayed in Examples 2 and 3.

### Example 2.

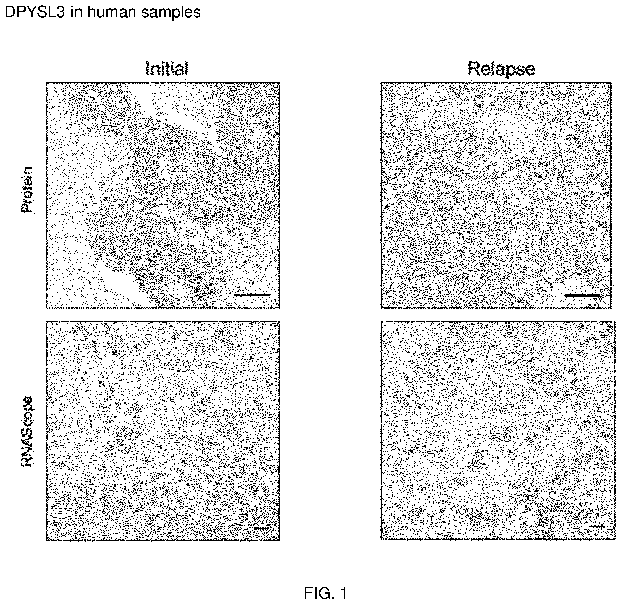

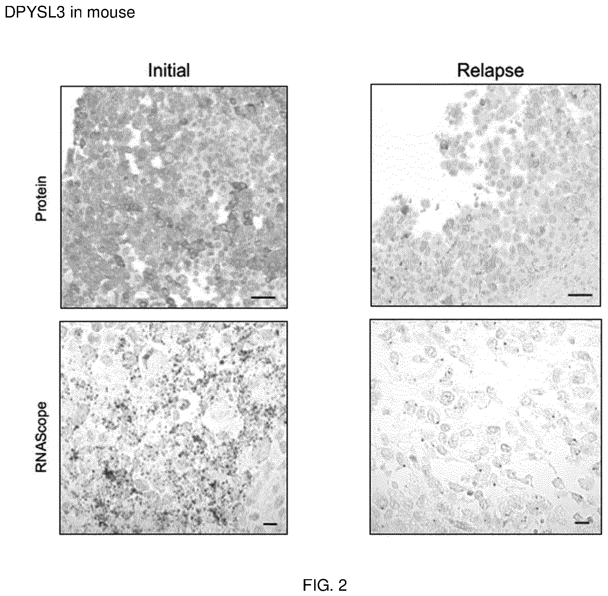

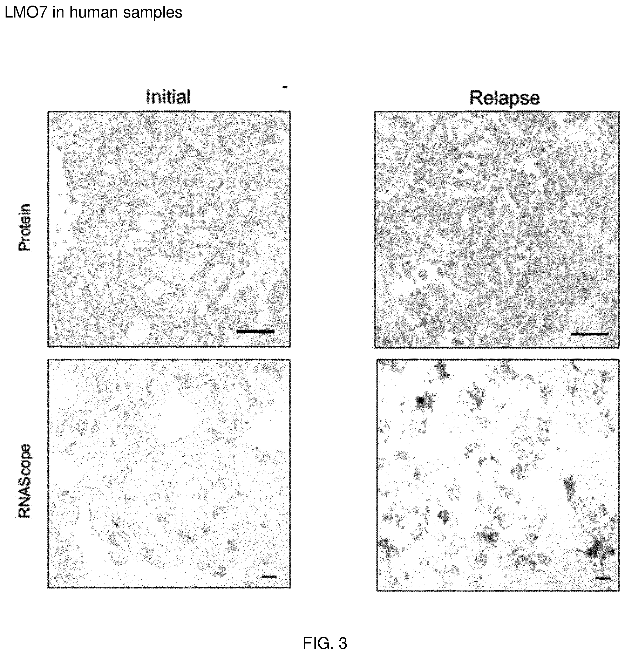

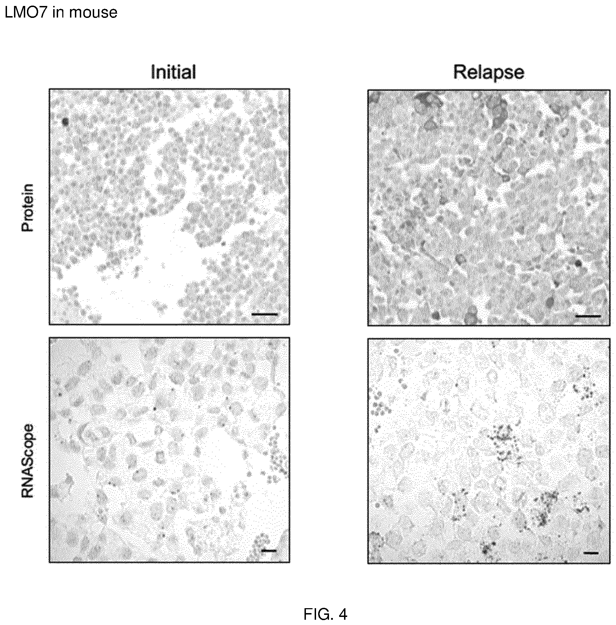

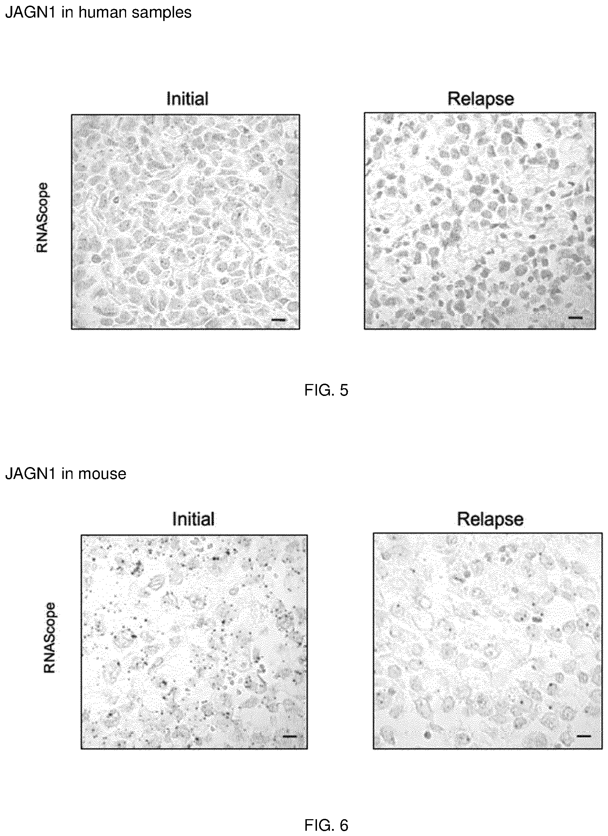

A validation of candidate genes *in situ* in experimental models of relapse was performed in the H2030-BrM lung cancer model by evaluation by RNAscope for LMO7, DPYSL3 JAGN1, and also with antibodies for LMO7 and DPYSL3. Corresponding results are displayed in Figures 1-6.

### Example 3.

Validation of biomarkers of relapse in human samples.

Analysis of rare matched human brain metastasis obtained from neurosurgery after an initial diagnosis (first surgery), and the one generated after some time after relapse post surgery (relapse post-Sx, when the local relapse appears), was performed in samples from 8 patients.

The markers previously validated in mouse models (i.e. *LMO7, DPYSL3, JAGN1)* were tested in matched human brain metastasis pre- and post-relapse.

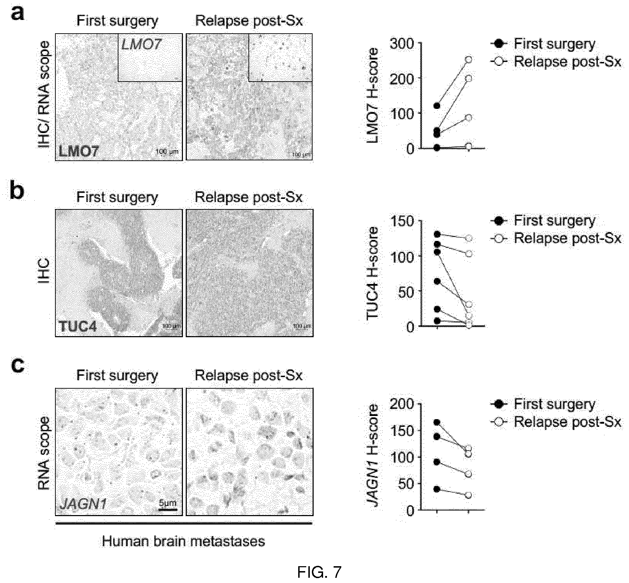

Results obtained are displayed in Figure 7. Basically, it was confirmed that LMO7 (a), DPYSL3 (also known as TUC4) (b) and *JAGN1* (c) followed and reproduced the data on experimental models. I.e. LMO7 is upregulated in relapse, while DPYSL3 and *JAGN1* are downregulated. While JAGN1 is only validated at RNA level (as corresponding antibodies were not functional), LMO7 and DPYSL3 have been validated at the protein level (as well as RNA).

Upon analysis of this samples it was concluded that LMO7 is upregulated, as predicted from experimental models, in relapse samples in 50% of the cases. While DPYSL3 and JAGN1 are downregulated, as predicted from experimental models, in relapse samples in 75% of the cases.

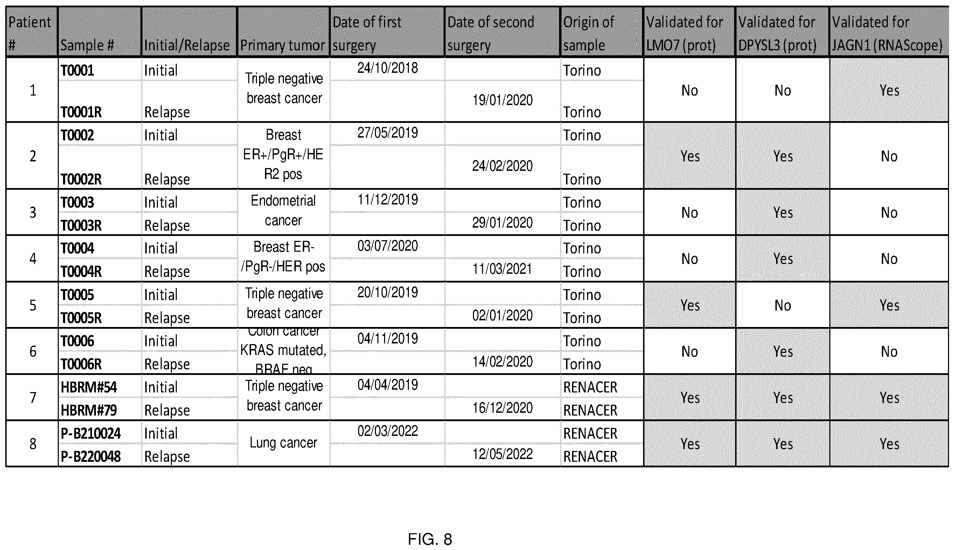

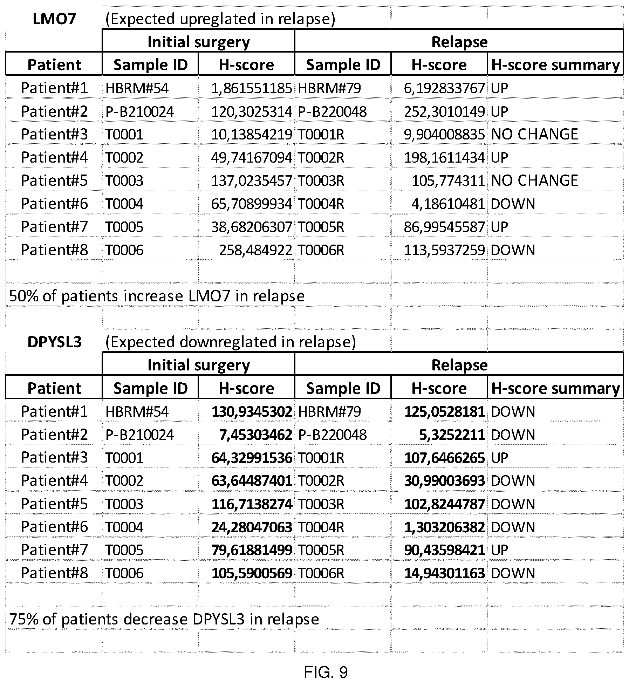

A table displaying the details of the 8 patient samples made use of is displayed in Figure 8. Results corresponding to LMO7 and DPYSL3 are displayed in Tables of Figures 9a and 9b, respectively.

As it can be observed, 4 out of the 8 patients whose samples have been analyzed, display alteration expression levels of both DPYSL3 and LMO7.

## Claims

1. A method for the prognosis of brain metastasis relapse in a subject, wherein the method comprises analyzing the expression levels of at least genes LMO7 and/or DPYSL3 in an isolated sample from the subject.

2. The method according to claim 1 wherein the expression levels of genes LMO7 and/or DPYSL3 are compared with those of reference populations with and/ or without relapse, and wherein an increased expression of gene LMO7 and a decreased expression of gene DPYSL3 when compared with a non-relapse reference population, is associated with a higher risk of relapse.

3. The method according to any of claims 1 or 2, further comprising analyzing the expression levels of gene JAGN1, and comparison of the corresponding expression levels with those of reference populations with and/or without relapse, wherein the decreased expression of gene JAGN1, when compared with a non-relapse reference population, is associated with a higher risk of relapse.

4. The method according to any of claims 1 to 3, further comprising analyzing the expression of one or more additional genes, and/ or of one or more reference genes in the sample.

5. The method according to claim 4, wherein the reference gene is selected from actin, beta-2 microglobulin, cytochrome C1, EMG1 N1 -specific pseudouridine methyltransferase, glyceraldehyde-3-phosphate dehydrogenase, TATA-box binding protein, topoisomerase (DNA) I, tyrosine 3- monooxygenase/tryptophan 5-monooxygenase activation protein zeta and combinations thereof, preferably is actin, beta-2 microglobulin or both.

6. The method according to any one of claims 1 to 5, wherein the subject has been subjected to treatment of the brain metastasis, preferably by brain surgery.

7. The method according to any one of claims 1 to 6, wherein the gene expression analysis is made by qPCR, preferably by detection of mRNA levels by qPCR, preferably by RT-qPCR, by *in situ* hybridization, preferably RNAscope, and/ or by protein detection preferably with antibodies.

8. The method according to any one of claims 1 to 7, wherein the sample is a brain sample obtained by brain surgery of a brain metastasis.

9. The method according to any one of claims 1 to 8, wherein the sample is a cDNA sample, preferably a cDNA obtained from an RNA sample.

10. The method according to any one of claims 1 to 9, wherein the subject is human, preferably a human with brain metastasis from a primary cancer selected from lung cancer, melanoma, colorectal cancer or breast cancer.

11. A kit comprising means for analyzing the expression levels of genes LMO7 and/or DPYSL3 in a sample.

12. The kit of claim 11 further comprising means for analyzing the expression levels of gene JAGN1, and/or one or more additional genes, and/ or of one or more reference genes in the sample.

13. The kit according to any one of claims 11 or 12, wherein the means for analyzing expression level of a gene involves inclusion of at least a pair of primers for any such gene and/ or of at least one specific probe for any such gene.

14. The kit according to any one of claims 10 to 13, further comprising at least one of the following: a DNA polymerase, a nucleotide mix, a buffer, DNase-free water, or any combination thereof.

15. Use of the method according to any one of claims 1 to 10, or of the kit according to any one of claims 11 to 14 for the prognosis of brain metastasis relapse.
